# EUROPEAN PATENT APPLICATION

(11) **EP 3 127 480 A1**
(43) Date of publication of application: **08.02.2017**
(21) Application number: 15772944.3
(22) Date of filing: 31.03.2015
(51) Int. Cl.: A61B 5/1455

(54) **BIOLOGICAL MEASUREMENT APPARATUS AND BIOLOGICAL MEASUREMENT METHOD**

(30) Priority: 04.04.2014 JP 2014077587
(71) Applicant: Seiko Epson Corporation, Tokyo 160-881 (JP)
(72) Inventor: IKEBE, Tomo, Suwa-shi, Nagano 392-8502 (JP)
(74) Representative: Hoffmann Eitle
(86) International application number: PCT/JP2015/001849
(87) International publication number: WO 2015/151508

(57) **Abstract**

To provide a biological measurement apparatus that accurately measures component concentration of a living organism.

The biological measurement apparatus includes a first detecting section 7 including a first infrared sensor 11 that radiates an infrared ray 26 on an arm 4, receives the infrared ray 26 reflected on the arm 4, and outputs light intensity data and a first force sensor 12 that detects a contact force between the first infrared sensor 11 and the arm 4, a second detecting section 8 including a second infrared sensor 13 and a second force sensor 14, a holding section 22 that holds the first detecting section 7 and the second detecting section 8 such that the first infrared sensor 11 and the second infrared sensor 13 are in contact with the arm 4, a pressing-force determining section that compares the force with a determination value and determines whether the force is within a predetermined range, and a component-concentration calculating section that calculates a blood sugar level using the light intensity data at the time when the pressing-force determining section determines that the contact force is within the predetermined range.

## Description

### Technical Field

The present invention relates to a biological measurement apparatus and a biological measurement method.

### Background Art

There has been examined a method of measuring a blood sugar level, blood pressure, and a pulse rate of a human body using a near infrared ray or an infrared ray. For example, PTL 1 discloses a measuring apparatus for measuring a blood sugar level using an ATR method (Attenuated Total Reflection). According to PTL 1, a glass plate, on one surface of which a reflection film is set, is prepared. A surface without the reflection film in the glass plate is closely attached to a skin of a living organism. An infrared ray is radiated from one side surface of the glass plate. The infrared ray repeats reflection between the surface closely attached to the skin and the reflection film and reaches the other side surface of the glass plate. Light intensity of the infrared ray reached the other side surface is detected by a sensor.

The detected infrared ray is split to obtain spectral data. Capillaries are present in the skin of the living organism. A part of the infrared ray radiated on the surface of the skin is absorbed by the capillaries. There is a correlation between the infrared ray absorbed at a specific wavelength and a blood component. Therefore, there is a correlation between light intensity at a detected specific wavelength and blood component concentration of a tested living organism. Consequently, it is possible to detect, for example, blood-glucose concentration using the ATR method.

PTL 2 discloses a measuring apparatus in which an optical sensor is set in a belt wound on an arm. According to PTL 2, the belt is set in a measuring apparatus main body. The belt is wound on an arm of a human body to fix the measuring apparatus. The optical sensor is set on a surface on the arm side of the belt. The length of the belt wound on the arm is adjusted and the optical sensor is closely attached to the skin to use the measuring apparatus.

### Citation List

### Patent Literature

PTL 1: JP-A-11-188009
PTL 2: JP-A-2000-254105

### Summary of Invention

### Technical Problem

In the ATR method described in PTL 1, when a sensor section is not closely attached to the skin of the living organism, a part of the infrared ray is not absorbed by the capillaries. At this point, since light intensity data does not reflect a state of the living organism, accurate blood component concentration cannot be detected. In the measuring apparatus described in PTL 2, when the belt loosens, a gap is formed between the optical sensor and the arm. Light intensity detected by the optical sensor fluctuates. There is a demand for a biological measurement apparatus that can accurately measure component concentration of a living organism utilizing only light intensity data at the time when detected light intensity data reflects a state of the living organism.

### Solution to Problem

The present invention has been devised in order to solve the problems and can be realized as the following forms or application examples.

### [Application Example 1]

A biological measurement apparatus according to this application example includes: a plurality of detecting sections including an infrared sensor section that radiates an infrared ray on a living organism, receives the infrared ray reflected or scattered on the living organism, and outputs light intensity data and a contact-force sensor section that detects a contact force between the infrared sensor section and the living organism; a holding section that holds the detecting section such that the infrared sensor section is in contact with the living organism; a determining section that compares the contact force with a determination value and determines whether the contact force is within a predetermined range; and a component-concentration calculating section that calculates component concentration of the living organism using the light intensity data at the time when the determining section determines that the contact force is within the predetermined range.

According to this application example, the biological measurement apparatus includes the plurality of detecting sections, the holding section, and the determining section. The detecting sections include the infrared sensor section and the contact-force sensor section. The holding section holds the detecting section such that the infrared sensor section is in contact with the living organism. The infrared sensor section radiates the infrared ray on the living organism. The infrared sensor section receives the infrared ray reflected or scattered on the living organism and outputs the light intensity data. The contact-force sensor section detects the contact force between the infrared sensor section and the living organism. The determining section compares the contact force with the determination value and determines whether the contact force is within the predetermined range.

The infrared ray is not absorbed by the living organism when the infrared sensor section is away from the living organism. Therefore, the infrared sensor section cannot accurately receive the infrared ray that reflects biological information. When the infrared sensor section excessively presses the living organism, a blood vessel of the living organism is pressed and a blood flow near the surface of the living organism is hindered. Consequently, since the living organism changes to a state unsuitable for measurement, the light intensity data received by the infrared sensor section changes to data that does not correctly reflects the state of the living organism.

The determining section determines a state in which the infrared sensor section presses the living organism. The component-concentration calculating section calculates the component concentration of the living organism utilizing data at the time when the light intensity data output by the infrared sensor section reflects the state of the living organism and not utilizing data at the time when the light intensity data does not reflect the state of the living organism. Therefore, the biological measurement apparatus can accurately measure the component concentration of the living organism utilizing only the light intensity data that reflects the state of the living organism among the light intensity data output by a plurality of the infrared sensor sections.

### [Application Example 2]

In the biological measurement apparatus according to the application example described above, the biological measurement apparatus includes an average calculating section that calculates an average of the component concentration calculated from the light intensity data output by a plurality of the infrared sensor sections.

According to this application example, the plurality of infrared sensor sections are set in the biological measurement apparatus. The infrared sensor sections output the light intensity data. The average calculating section calculates an average of the light intensity data output by the plurality of infrared sensor sections. Therefore, it is possible to suppress the influence of disturbances and measure highly reliable component concentration.

### [Application Example 3]

In the biological measurement apparatus according to the application example described above, a plurality of the infrared sensor sections are sequentially driven.

According to this application example, the infrared sensor sections are sequentially driven. Consequently, compared with when a part of the plurality of infrared sensor sections are simultaneously driven, it is possible to reduce a peak value of electric power consumed by the driving. Therefore, since an ability to supply the electric power can be reduced, it is possible to reduce the capacity of a power supply.

### [Application Example 4]

In the biological measurement apparatus according to the application example described above, the detecting section includes a plurality of the contact-force sensor sections.

According to this application example, the detecting section includes the infrared sensor section and the plurality of contact-force sensor sections. The determining section compares contact forces output by the plurality of contact-force sensor sections with the determination value. When the infrared sensor section tilts with respect to the living organism, a difference occurs among contact forces received by the plurality of contact-force sensor sections from an arm. Consequently, it is possible to also detect a state in which the infrared sensor section tilts with respect to the living organism. The light intensity data output by the infrared sensor section in the state in which the infrared sensor section tilts with respect to the living organism is not used. Therefore, it is possible to use the light intensity data of reflected light detected by the infrared sensor section when the infrared sensor section is in a state of a correct posture without tilting with respect to the living organism. As a result, it is possible to accurately detect information concerning the living organism.

### [Application Example 5]

A biological measurement apparatus according to this application example includes: a plurality of detecting sections including an infrared sensor section that radiates an infrared ray on a living organism, receives the infrared ray reflected or scattered on the living organism, and outputs light intensity data and a contact-force sensor section that detects a contact force between the infrared sensor section and the living organism; a holding section that holds the detecting section such that the infrared sensor section is in contact with the living organism; a determining section that compares the contact force with a determination value and determines whether the contact force is within a predetermined range; an infrared-sensor control section that performs control for driving the infrared sensor section when the contact force is within a predetermined range; and a component-concentration calculating section that calculates component concentration of the living organism using the light intensity data.

According to this application example, the determining section determines a state in which the infrared sensor section presses the living organism. The infrared-sensor control section drives the infrared sensor section when a contact force of the infrared sensor section pressing the living organism is within the predetermined range. Therefore, the infrared-sensor control section drives the infrared sensor section only when the infrared sensor section can output appropriate light intensity data. Therefore, the biological measurement apparatus can accurately measure the component concentration of the living organism utilizing only the light intensity data that reflects the state of the living organism among the light intensity data output by a plurality of the infrared sensor sections. Further, since electric power for driving the infrared sensor section is not wasted, it is possible to drive the infrared sensor section while saving resources.

### [Application Example 6]

A biological measurement method according to this application example includes: pressing a plurality of infrared sensor sections against a living organism; detecting contact forces between the infrared sensor sections and the living organism; driving the infrared sensor sections, the contact forces of which are within a predetermined range; the infrared sensor sections radiating an infrared ray on the living organism, receiving the infrared ray reflected or scattered on the living organism, and outputting light intensity data; and calculating component concentration of the living organism from the light intensity data.

According to this application example, the plurality of infrared sensor sections are pressed against the living organism. The infrared sensor sections radiate the infrared ray on the living organism, receive the infrared ray reflected or scattered on the living organism, and output the light intensity data. The component concentration of the living organism is calculated using the light intensity data. The contact forces between the infrared sensor sections and the living organism are detected. The infrared sensor sections are driven when the contact forces are within the predetermined range. Therefore, the infrared sensor sections detect the light intensity data of reflected light of the infrared ray only when the infrared sensor sections can accurately detect the light intensity data. As a result, it is possible to accurately detect light intensity of the infrared ray reflected from the living organism.

### Brief Description of the Drawings

[Fig. 1] Fig. 1 relates to a first embodiment, wherein Fig. 1(a) is a schematic perspective view showing the configuration of a biological measurement apparatus, Fig. 1(b) is a schematic front view showing the configuration of the biological measurement apparatus, and Fig. 1(c) is a schematic side view showing the configuration of the biological measurement apparatus.
[Fig. 2] Fig. 2(a) is a main part schematic enlarged side view showing a main body section of the biological measurement apparatus, Fig. 2(b) is a main part schematic view showing the structure of an infrared sensor, and Fig. 2(c) is a main part schematic enlarged view showing the structure of a force sensor.
[Fig. 3] Fig. 3 is an electric control block diagram of the biological measurement apparatus.
[Fig. 4] Fig. 4 is a flowchart of a biological measurement method.
[Fig. 5] Fig. 5 is a diagram for explaining the biological measurement method.
[Fig. 6] Fig. 6 is a diagram for explaining the biological measurement method.
[Fig. 7] Fig. 7 is a diagram for explaining the biological measurement method.
[Fig. 8] Fig. 8 is a main part schematic enlarged view showing the structure of a force sensor according to a second embodiment.
[Fig. 9] Fig. 9 relates to a third embodiment, wherein Fig. 9 (a) is a main part schematic enlarged view showing the structure of a main body section and Fig. 9(b) is a main part schematic enlarged view showing the structure of a belt section.
[Fig. 10] Figs. 10(a) to 10(c) are schematic diagrams for explaining a biological measurement method.
[Fig. 11] Fig. 11 is a schematic diagram showing the disposition of a detecting section.
[Fig. 12] Fig. 12 is a schematic diagram showing the disposition of the detecting section.
[Fig. 13] Fig. 13 is a time chart showing driving timings of infrared sensors according to a fourth embodiment.

### Description of Embodiments

In embodiments, characteristic examples of a biological measurement apparatus and a biological measurement method for measuring a blood sugar level in a non-invasive manner using the biological measurement apparatus are explained with reference to the drawings. The embodiments are explained below with reference to the drawings. Note that members in the drawings are shown with scales varied for the respective members to show the members in sizes recognizable on the drawings.

### (First Embodiment)

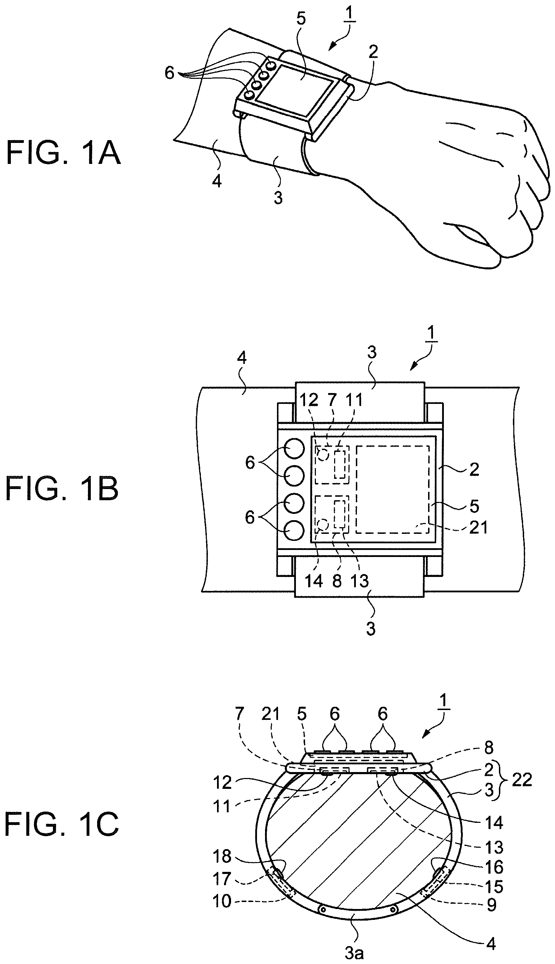

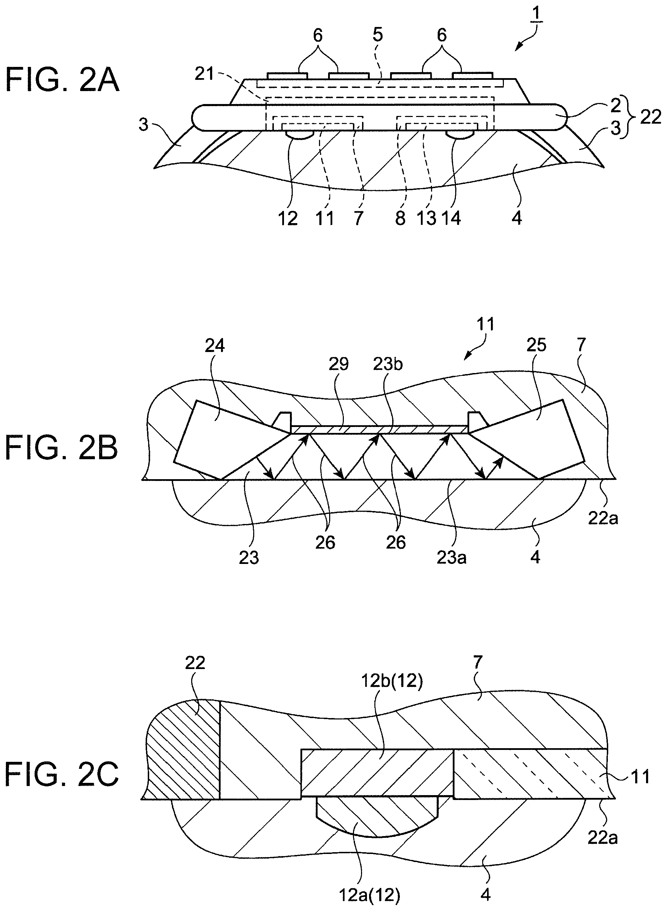

A biological measurement apparatus according to a first embodiment is explained with reference to Fig. 1 to Fig. 3. Fig. 1(a) is a schematic perspective view showing the configuration of the biological measurement apparatus. Fig. 1(b) is a schematic front view showing the configuration of the biological measurement apparatus. Fig. 1(c) is a schematic side view showing the configuration of the biological measurement apparatus. Fig. 2 (a) is a main part schematic enlarged side view showing a main body section of the biological measurement apparatus.

As shown in Fig. 1 and Fig. 2(a), a biological measurement apparatus 1 includes a main body section 2 functioning as a holding section and a belt section 3 functioning as a holding section set in the main body section 2. The biological measurement apparatus 1 is formed in a cylindrical shape by the main body section 2 and the belt section 3. An arm 4 serving as a living organism of a human body is inserted into a place formed in the cylindrical shape. That is, the biological measurement apparatus 1 is set on the arm 4 like a wristwatch and used.

A display device 5 and an input device 6 are set on a surface on the opposite side of the arm 4 in the main body section 2. The display device 5 is a part that displays information such as a measured blood sugar level. As the display device 5, a liquid crystal display device, an organic EL (Electro Luminescence) device, and the like can be used. The input device 6 is configured by a plurality of switches or the like. An operator is capable of performing various kinds of setting by operating the input device 6.

A first detecting section 7 functioning as a detecting section and a second detecting section 8 functioning as a detecting section are set on a surface on a side facing the arm 4 in the main body section 2. Further, a third detecting section 9 functioning as a detecting section and a fourth detecting section 10 functioning as a detecting section are set on a surface on a side facing the arm 4 in the belt section 3. A first infrared sensor 11 functioning as an infrared sensor section and a first force sensor 12 functioning as a contact-force sensor section are set in the first detecting section 7. A second infrared sensor 13 functioning as an infrared sensor section and a second force sensor 14 functioning as a contact-force sensor section are set in the second detecting section 8.

A third infrared sensor 15 functioning as an infrared sensor section and a third force sensor 16 functioning as a contact-force sensor section are set in the third detecting section 9. A fourth infrared sensor 17 functioning as an infrared sensor section and a fourth force sensor 18 functioning as a contact-force sensor section are set in the fourth detecting section 10. A control section 21 is set on the inside of the main body section 2. The control section 21 controls the display device 5, the input device 6, and the first detecting section 7 to the fourth detecting section 10. Besides, the main body section 2 includes a battery and a buzzer not shown in the figure.

An adjusting section 3a is set in the belt section 3. The adjusting section 3a is capable of adjusting the length of the belt section 3. The operator operates the adjusting section 3a after wearing the biological measurement apparatus 1 on the arm 4. The operator adjusts the length of the belt section 3 such that the first detecting section 7 to the fourth detecting section 10 are in contact with the arm 4. The belt section 3 is made of metal and is formed in structure in which a plurality of leaf springs are coupled. Consequently, the belt section 3 extends and contracts. A holding section 22 is configured by the main body section 2 and the belt section 3. The first infrared sensor 11 to the fourth infrared sensor 17 and the first force sensor 12 to the fourth force sensor 18 are held by the holding section 22 to be in contact with the arm 4.

Fig. 2(b) is a main part schematic diagram showing the structure of the infrared sensor. As shown in Fig. 2 (b), the first infrared sensor 11 includes a tabular prism 23. An opposed pair of side surfaces of the prism 23 is slopes. A first light emitting device 24 is set on one slope. A first light receiving device 25 is set on the other slope. The material of the prism 23 only has to be a material that transmits an infrared ray. For example, ZnSe, Ge, and Si can be used.

The first light emitting device 24 includes an LED (Light Emitting Diode). The light emitting device 24 radiates an infrared ray 26 toward the inside of the prism 23. The first light receiving device 25 includes a spectrometer and a photodiode. The first light receiving device 25 splits the infrared ray 26 into a predetermined wavelength and converts light intensities of wavelengths into electric signals. The spectrometer is configured by combining a diffraction grating, a concave mirror, and a slit. The spectrometer rotates the mirror and splits the infrared ray 26 such that light having the predetermined wavelength passes through the slit. The infrared ray 26 passed through the slit is converted into an electric signal by the photodiode.

A surface in contact with the arm 4 in the prism 23 is represented as a first surface 23a. A surface opposed to the first surface 23a is represented as a second surface 23b. The first surface 23a and the second surface 23b are disposed in parallel. A reflection film 29 is set on the second surface 23b. As the reflection film 29, it is desirable to use metal having high reflectance such as silver. The infrared ray 26 radiated by the first light emitting device 24 travels toward the first surface 23a. The infrared ray 26 irradiates the surface of the arm 4 on the first surface 23a. The infrared ray 26 is reflected on the surface of the arm 4. The reflected infrared ray 26 travels toward the reflection film 29 and is reflected on the reflection film 29. In this way, the infrared ray 26 repeats the reflection between the surface of the arm 4 on the first surface 23a and the second surface 23b and travels toward the first light receiving device 25.

A part of the infrared ray 26 having the predetermined wavelength is absorbed by capillaries in the arm 4 every time the infrared ray 26 is reflected on the first surface 23a. Consequently, light intensity at the predetermined wavelength of the infrared ray 26 decreases. The infrared ray 26 reached the first light receiving device 25 is split and converted into an electric signal. The electric signal is a signal corresponding to light intensity data. That is, the first infrared sensor 11 radiates the infrared ray 26 on the arm 4, receives the infrared ray 26 reflected on the arm 4, and outputs light intensity data. The second infrared sensor 13 to the fourth infrared sensor 17 have structure same as the structure of the first infrared sensor 11 and has a function same as the function of the first infrared sensor 11.

When a surface on a side facing the arm 4 in the holding section 22 is represented as a contact surface 22a, the contact surface 22a is set on a plane substantially the same as the first surface 23a. Therefore, usually, the holding section 22 holds the first infrared sensor 11 to the fourth infrared sensor 17 such that the first surface 23a is in contact with the arm 4.

Fig. 2(c) is a main part schematic enlarged view showing the structure of the force sensor. As shown in Fig. 2(c), the first force sensor 12 includes a sensor main body section 12b and a pressing protrusion 12a functioning as a protrusion section. A piezoelectric element is incorporated in the sensor main body section 12b. The piezoelectric element is held between the sensor main body section 12b and the pressing protrusion 12a. When the pressing protrusion 12a is pressed toward the sensor main body section 12b, force is applied to the piezoelectric element. The piezoelectric element generates and outputs a voltage corresponding to a differential value of the force. Therefore, it is possible to detect, using the voltage output by the piezoelectric element, pressure applied to the first force sensor 12.

The first infrared sensor 11 and the first force sensor 12 are held by the holding section 22 and are set in contact with the arm 4. The first infrared sensor 11 and the first force sensor 12 are set in places close to each other. Therefore, pressure applied to the first infrared sensor 11 by the arm 4 and pressure applied to the first force sensor 12 by the arm 4 are substantially the same pressure. It is possible to detect a pressing force applied to the first infrared sensor 11 by detecting a pressing force applied to the first force sensor 12. Therefore, the first force sensor 12 can detect forces of the first infrared sensor 11 and the arm 4 pressing each other.

The pressing protrusion 12a projects to the arm 4 side from the contact surface 22a of the holding section 22. The contact surface 22a is a surface substantially the same as the first surface 23a of the prism 23. Therefore, the pressing protrusion 12a projects to the arm 4 side from the first infrared sensor 11. Consequently, the pressing protrusion 12a can be set in contact with the arm 4 even in a state in which the first infrared sensor 11 and the arm 4 are apart from each other. Therefore, the first force sensor 12 can surely detect whether the first infrared sensor 11 and the arm 4 are in contact with each other. The second force sensor 14 to the fourth force sensor 18 have structure same as the structure of the first force sensor 12 and have a function same as the function of the first force sensor 12.

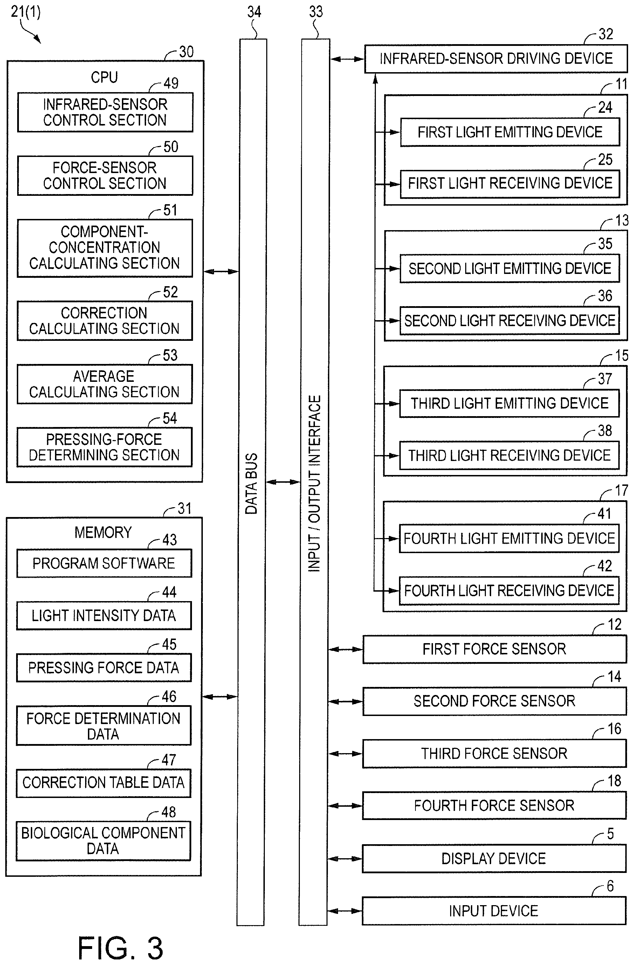

Fig. 3 is an electric control block diagram of the biological measurement apparatus. As shown in Fig. 3, the control section 21 of the biological measurement apparatus 1 includes a CPU 30 (an arithmetic processing device) that functions as a processor and performs various kinds of arithmetic processing and a memory 31 that stores various kinds of information.

An infrared-sensor driving device 32, the first force sensor 12 to the fourth force sensor 18, the display device 5, and the input device 6 are connected to the CPU 30 via an input/output interface 33 and a data bus 34. The infrared-sensor driving device 32 is a device that drives the first light emitting device 24 and the first light receiving device 25 of the first infrared sensor 11. Besides, the infrared-sensor driving device 32 drives a second light emitting device 35 and a second light receiving device 36 of the second infrared sensor 13. Besides, the infrared-sensor driving device 32 drives a third light emitting device 37 and a third light receiving device 38 of the third infrared sensor 15. Besides, the infrared-sensor driving device 32 drives a fourth light emitting device 41 and a fourth light receiving device 42 of the fourth infrared sensor 17.

The infrared-sensor driving device 32 receives an input of an instruction signal from the CPU 30 and lights the first light emitting device 24 to the fourth light emitting device 41. The infrared-sensor driving device 32 drives the first light receiving device 25 to the fourth light receiving device 42 and inputs light intensities at wavelengths output by the first light receiving device 25 to the fourth light receiving device 42. The infrared-sensor driving device 32 outputs light intensity data including a measured spectrum or the like to the CPU 30.

The input device 6 includes, besides the plurality of switches, an interface for receiving inputs from various kinds of setting information from an external apparatus. The biological measurement apparatus 1 can receive, via the input device 6, from the external apparatus, inputs of data and the like used when a biological component is extracted.

The memory 31 is a concept including a semiconductor memory such as a RAM or a ROM and an external storage device such as an external hard disk or a CD-ROM. In terms of a function, a storage region for storing program software 43 describing a control procedure of an operation in the biological measurement apparatus 1 is set. Further, a storage region for storing light intensity data 44, which is data of spectra output by the first infrared sensor 11 to the fourth infrared sensor 17, is also set. Besides, a storage region for storing pressing force data 45, which is data of forces of the first infrared sensor 11 to the fourth infrared sensor 17 and the arm 4 pressing each other detected by the first forth sensor 12 to the fourth force sensor 18, is set.

Besides, a storage region for storing force determination data 46, which is data at the time when the CPU 30 determines a pressing force using the pressing force data 45, is set. Besides, a storage region for storing correction table data 47, which is data used when the CPU 30 corrects the light intensity data 44 using the pressing force data 45, is set. The correction table data 47 is data indicating a relation of a correction amount of the light intensity data 44 with pressing forces of the arm 4 pressing the first infrared sensor 11 to the fourth infrared sensor 17. Besides, a storage region for storing biological component data 48, which is data used when the CPU 30 calculates component concentration of blood and measured component concentration data, is set. Further, a storage region functioning as a work area, a temporary file, and the like for the CPU 30 and other various storage regions are set.

The CPU 30 performs, according to the program software 43 stored in the memory 31, control for detecting a blood component using data output by the first infrared sensor 11 to the fourth infrared sensor 17. As a specific function realizing section, the CPU 30 includes an infrared-sensor control section 49 that performs an instruction for causing the infrared-sensor driving device 32 to drive the first infrared sensor 11 to the fourth infrared sensor 17. Further, the CPU 30 includes a force-sensor control section 50 that performs an instruction for causing the first force sensor 12 to the fourth force sensor 18 to detect pressing forces between the first infrared sensor 11 to the fourth infrared sensor 17 and the arm 4. Further, the CPU 30 includes a component-concentration calculating section 51 that calculates blood component concentration using the light intensity data 44 detected by the first infrared sensor 11 to the fourth infrared sensor 17.

Further, the CPU 30 includes a correction calculating section 52 functioning as a correcting section that performs calculation for correcting the light intensity data 44 using the pressing force data 45. Further, the CPU 30 includes an average calculating section 53 that calculates the biological component data 48 and an average of blood component concentration. Further, the CPU 30 includes a pressing-force determining section 54 functioning as a determining section that determines, using the pressing force data 45 and the force determination data 46, whether the light intensity data 44 is used.

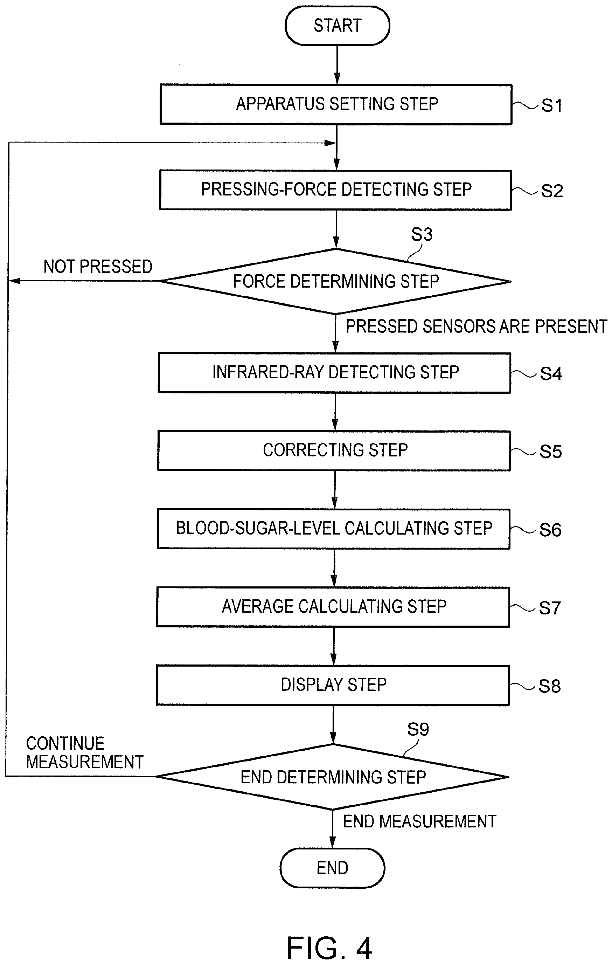

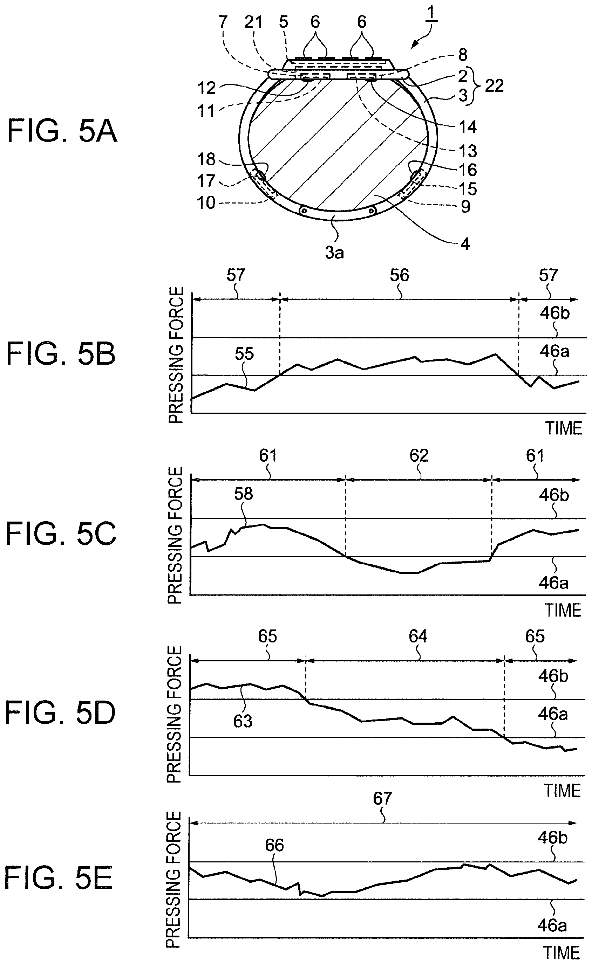

A biological measurement method for measuring blood component concentration using the biological measurement apparatus 1 is explained with reference to Fig. 4 to Fig. 7. Fig. 4 is a flowchart of the biological measurement method. Fig. 5 to Fig. 7 are diagrams for explaining the biological measurement method.

In the flowchart of Fig. 4, step S1 is equivalent to an apparatus setting step and is a step for setting the biological measurement apparatus 1 in the arm 4. In this step, the biological measurement apparatus 1 is set such that the first detecting section 7 to the fourth detecting section 10 press the arm 4. Consequently, the holding section 22 sets the first infrared sensor 11 to the fourth infrared sensor 17 in contact with the arm 4. Processing of the biological measurement method shifts to step S2. Step S2 is equivalent to a pressing-force detecting step. This step is a step in which the first force sensor 12 to the fourth force sensor 18 detect pressing forces, which are forces of the first infrared sensor 11 to the fourth infrared sensor 17 and the arm 4 pressing each other. The processing shifts to step S3. Step S3 is equivalent to a force determining step and is a step for determining whether the detected pressing forces are within a determination value. When it is determined that several pressing forces among the pressing forces are within the determination value and any one of the first infrared sensor 11 to the fourth infrared sensor 17 and the arm 4 press each other, the processing shifts to step S4. When it is determined that all of the pressing forces are not within the determination value and the first infrared sensor 11 to the fourth infrared sensor 17 and the arm 4 do not press each other, the processing shifts to step S2.

Step S4 is equivalent to an infrared-ray detecting step. In this step, the infrared-sensor driving device 32 drives the infrared sensor that presses the arm 4 among the first infrared sensor 11 to the fourth infrared sensor 17. The driven infrared sensor radiates the infrared ray 26 on the arm 4. This step is a step in which the first infrared sensor 11 to the fourth infrared sensor 17 receive the infrared ray 26 reflected on the arm 4 and output the light intensity data 44, which is a spectrum of reflected light. The processing shifts to step S5. Step S5 is equivalent to a correcting step. This step is a step for correcting the light intensity data 44 using the pressing force data 45 and the correction table data 47. The processing shifts to step S6. Step S6 is equivalent to a blood-sugar-level calculating step. This step is a step for calculating a blood sugar level from the corrected light intensity data 44. The processing shifts to step S7.

Step S7 is equivalent to an average calculating step. This step is a step for calculating an average of the blood sugar level. The processing shifts to step S8. Step S8 is equivalent to a display step. This step is a step for displaying the calculated average of the blood sugar level on the display device 5. The processing shifts to step S9. Step S9 is equivalent to an end determining step and is a step for determining whether the measurement is ended or continued. When the measurement is continued, the processing shifts to step S2. When the measurement is ended, the processing of the biological measurement method is ended.

The biological measurement method is explained in detail with reference to Fig. 5 to Fig. 7 while associating the figures with the steps shown in Fig. 4. Fig. 5 (a) is a diagram corresponding to the apparatus setting step in step S1. As shown in Fig. 5(a), the operator wears the biological measurement apparatus 1 on the arm 4. The operator operates the adjusting section 3a to adjust the length of the belt section 3. The operator presses the first detecting section 7 to the fourth detecting section 10 against the arm 4 and closely attaches the first detecting section 7 to the fourth detecting section 10 to the arm 4. The first infrared sensor 11 to the fourth infrared sensor 17 are set in contact with the arm 4 by the holding section 22. At this point, the operator adjusts the length of the belt section 3 to a degree in which the belt section 3 does not squeeze the arm 4 and blood in a blood vessel of the arm 4 smoothly flows.

Fig. 5(b) to Fig. 5(e) are diagrams corresponding to the pressing-force detecting step in step S2 and the force determining step in step S3. In the figures, the ordinate indicates the pressing force detected by the force sensor. The pressing force is larger on the upper side than the lower side in the figures. The abscissa indicates elapse of time. The time changes from the left to the right in the figures. In step S2, the force-sensor control section 50 outputs an instruction for instructing the first force sensor 12 to the fourth force sensor 18 to detect pressing forces. The first force sensor 12 to the fourth force sensor 18 cause the memory 31 to store pressing forces received from the arm 4 as the pressing force data 45. A first force transition line 55 shown in Fig. 5(b) indicates transition of the pressing force detected by the first force sensor 12.

The force determination data 46 is stored in the memory 31. The force determination data 46 includes a lower determination value 46a and an upper determination value 46b. The pressing-force determining section 54 compares the pressing force and the lower determination value 46a. When the pressing force is smaller than the lower determination value 46a, the pressing-force determining section 54 determines that the pressing force is insufficient. Further, the pressing-force determining section 54 compares the pressing force and the upper determination value 46b. When the pressing force is larger than the upper determination value 46b, the pressing-force determining section 54 determines that the pressing force is excessive.

When the pressing force is equal to or larger than the lower determination value 46a and equal to or smaller than the upper determination value 46b, the pressing-force determining section 54 determines that the pressing force is proper. Time when the pressing force is proper in the first force transition line 55 is referred to as first proper section 56. Time when the pressing force is insufficient or excessive in the first force transition line 55 is referred to as first improper section 57.

A second force transition line 58 shown in Fig. 5(c) indicates transition of the pressing force detected by the second force sensor 14. The pressing-force determining section 54 compares the pressing force with the lower determination value 46a and the upper determination value 46b. Time when the pressing force is proper in the second force transition line 58 is referred to as second proper section 61. Time when the pressing force is insufficient or excessive in the second force transition line 58 is referred to as second improper section 62.

A third force transition line 63 shown in Fig. 5 (d) indicates transition of the pressing force detected by the third force sensor 16. The pressing-force determining section 54 compares the pressing force with the lower determination value 46a and the upper determination value 46b. Time when the pressing force is proper in the third force transition line 63 is referred to as third proper section 64. Time when the pressing force is insufficient or excessive in the third force transition line 63 is referred to as third improper section 65.

The fourth force transition line 66 shown in Fig. 5(e) indicates transition of the pressing force detected by the fourth force sensor 18. The pressing-force determining section 54 compares the pressing force with the lower determination value 46a and the upper determination value 46b. Time when the pressing force is proper in the fourth force transition line 66 is referred to as fourth proper section 67. Time when the pressing force is insufficient or excessive in the fourth force transition line 66 is referred to as fourth improper section 66. A section corresponding to the fourth improper section is absent in the fourth force transition line 66 in the figure. Therefore, the fourth improper section is not displayed.

In the first improper section 57, the second improper section 62, the third improper section 65, and the fourth improper section, the detecting section may display "detection failure" on the display device 5. Further, the detecting section may emit sound from the buzzer to urge the operator to perform adjustment.

When all of the pressing forces detected by the first force sensor 12 to the fourth force sensor 18 are improper, step S2 and step S3 are repeated. When several pressing forces among the pressing forces detected by the first force sensor 12 to the fourth force sensor 18 are proper, the processing shifts to step S4.

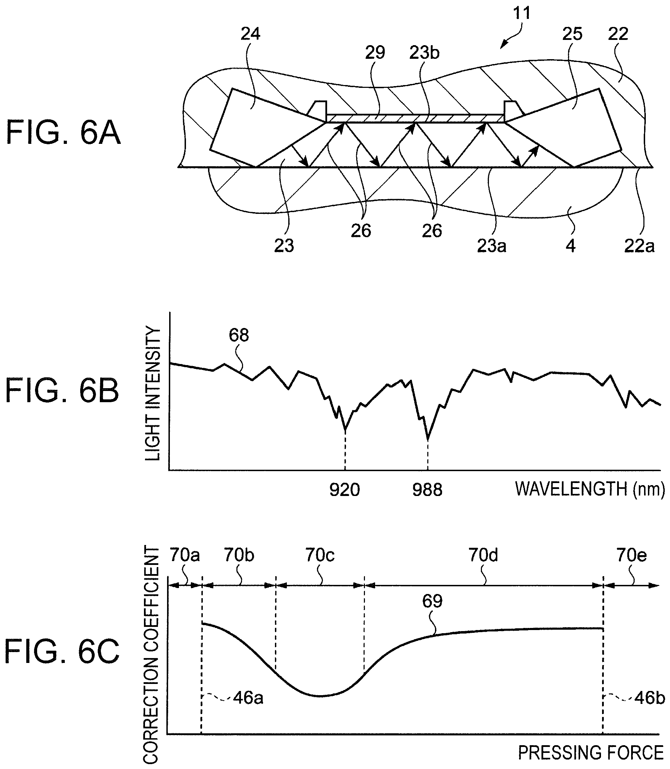

Fig. 6(a) and Fig. 6(b) are diagrams corresponding to the infrared-ray detecting step in step S4. In step S4, the infrared-sensor control section 49 outputs an instruction signal for driving the first infrared sensor 11 to the fourth infrared sensor 17 to the infrared-sensor driving device 32. The infrared-sensor control section 49 drives the infrared sensors corresponding to the force sensors, the detected pressing forces of which are proper. That is, when the pressing force detected by the first force sensor 12 is proper, the infrared-sensor control section 49 drives the first infrared sensor 11. When the pressing force detected by the second force sensor 14 is proper, the infrared-sensor control section 49 drives the second infrared sensor 13. When the pressing force detected by the third force sensor 16 is proper, the infrared-sensor control section 49 drives the third infrared sensor 15. When the pressing force detected by the fourth force sensor 18 is proper, the infrared-sensor control section 49 drives the fourth infrared sensor 17. That is, only when the force sensors are in the proper sections, the infrared-sensor control section 49 drives the infrared sensors corresponding to the force sensors.

In the first proper section 56, as shown in Fig. 6(a), the infrared-sensor driving device 32 lights the first light emitting device 24. The first light emitting device 24 radiates the infrared ray 26 on the prism 23. In the prism 23, the infrared ray 26 repeats reflection between the surface of the arm 4 closely attached to the first surface 23a and the second surface 23b and travels to the first light receiving device 25. The infrared ray 26 irradiates the surface of the arm 4 on the first surface 23a. The infrared ray 26 having a part of wavelengths is absorbed by the blood in the capillaries of the arm 4.

The first light receiving device 25 detects light intensity at each of the wavelengths. As a result, as shown in Fig. 6(b), a spectrum of the infrared ray 26 is output. The ordinate in the figure indicates the light intensity. The light intensity is higher on the upper side than the lower side in the figure. The abscissa indicates the wavelength. The wavelength is longer on the right side than the left side in the figure. A light intensity distribution line 68 indicates light intensities of the infrared ray 26 at the wavelengths. The light intensities are small in places of the wavelengths 920 nm and 988 nm in the light intensity distribution line 68. At the wavelengths, the infrared ray 26 is absorbed by the blood in the capillaries. As glucose concentration in the blood is higher, the infrared ray 26 is further absorbed.

Similarly, in the second proper section 61, the second infrared sensor 13 is driven and a spectrum of the infrared ray 26 is output. In the third proper section 64, the third infrared sensor 15 is driven and a spectrum of the infrared ray 26 is output. In the fourth proper section 67, the fourth infrared sensor 17 is driven and a spectrum of the infrared ray 26 is output.

Fig. 6 (c) is a diagram corresponding to the correcting step in step S5. In step S5, the correction calculating section 52 corrects the light intensity distribution line 68. The correction table data 47 is stored in the memory 31. A correction curve 69 shown in Fig. 6 (c) is a curve indicating a table of the correction table data 47. The correction table data 47 shows a relation between the pressing forces detected by the first force sensor 12 to the fourth force sensor 18 and correction amounts for correcting the spectra detected by the first infrared sensor 11 to the fourth infrared sensor 17. The correction curve 69 is a curve set on the basis of a relation between the pressing force and the light intensity detected by the first infrared sensor 11. In the figure, the ordinate indicates a correction coefficient. The correction coefficient is larger on the upper side than the lower side in the figure. The abscissa indicates the pressing force. The pressing force is larger on the right side than the left side in the figure. As the correction coefficient is larger, the correction amount is larger.

The correction curve 69 is divided into a first section 70a, a second section 70b, a third section 70c, a fourth section 70d, and a fifth section 70e and explained. The first section 70a is a section in which the pressing force is smaller than the lower determination value 46a and is a section in which the contact of the first surface 23a of the prism 23 and the arm 4 is unstable. Therefore, since the infrared-ray detecting step in step S4 is not performed, the correction curve 69 is not set.

In the second section 70b, the first infrared sensor 11 can detect the absorption of the infrared ray 26 by the blood in the capillaries. However, the second section 70b is a section in which the pressing force of the first infrared sensor 11 and the arm 4 pressing each other is small. The second section 70b is a section in which, as the pressing force is larger, the absorption of the infrared ray 26 by the blood in the capillaries is larger. In this section, as the pressing force is smaller, the correction coefficient is increased.

The third section 70c is a section in which the pressing force is proper and the first infrared sensor 11 can appropriately detect the absorption of the infrared ray 26 by the blood in the capillaries. In this section, the correction of the light intensity distribution line 68 is reduced.

The fourth section 70d is a section in which the first infrared sensor 11 can detect the absorption of the infrared ray 26 by the blood in the capillaries but the capillaries are pressed and the blood decreases. As the first infrared sensor 11 is further pressed by the arm 4, the blood decreases and the absorption of the infrared ray 26 decreases. Therefore, in this section, as the pressing force increases, the correction coefficient is increased.

The fifth section 70e is a section in which the first infrared sensor 11 unstably detects the absorption of the infrared ray 26 by the blood in the capillaries. Therefore, since the infrared-ray detecting step in step S4 is not performed, the correction curve 69 is not set.

In step S5, the correction calculating section 52 calculates the correction coefficient from the pressing force and the correction curve 69 and integrates the correction coefficient into the light intensity distribution line 68. When the operator moves the arm 4, relative positions of the arm 4 and the biological measurement apparatus 1 move. At this point, the pressing force of the first infrared sensor 11 and the arm 4 pressing each other fluctuates. The correction calculating section 52 corrects the light intensity distribution line 68 using the pressing force and the correction curve 69. Therefore, even when the pressing force fluctuates, it is possible to obtain the accurate light intensity distribution line 68. Further, concerning the spectra detected by the second infrared sensor 13 to the fourth infrared sensor 17, similarly, the correction calculating section 52 calculates the correction coefficient from the pressing force and the correction curve 69 and integrates the correction coefficient into the light intensity distribution line.

In the blood-sugar-level calculating step in step S6, the component-concentration calculating section 51 calculates a blood sugar level using a value obtained by correcting the light intensity distribution line 68. A light intensity-to-blood sugar level correlation table indicating a relation between light intensity and a blood sugar level at a predetermined wavelength is stored in the biological component data 48. The component-concentration calculating section 51 calculates a blood sugar level using the light intensity-to-blood sugar level correlation table and the light intensity distribution line 68. The blood sugar level at this point indicates a glucose amount in the blood. Note that a PLS regression analysis method (Partial Least Squares Regression) may be applied to the calculation of the blood sugar level to more accurately calculate the blood sugar level.

A calculation result of the blood sugar level is stored in the memory 31 as the biological component data 48. After ending the measurement, the biological measurement apparatus 1 can transmit data of the blood sugar level to an external apparatus such as a personal computer. Therefore, it is possible to analyze transition of the blood sugar level according to a program set in the personal computer.

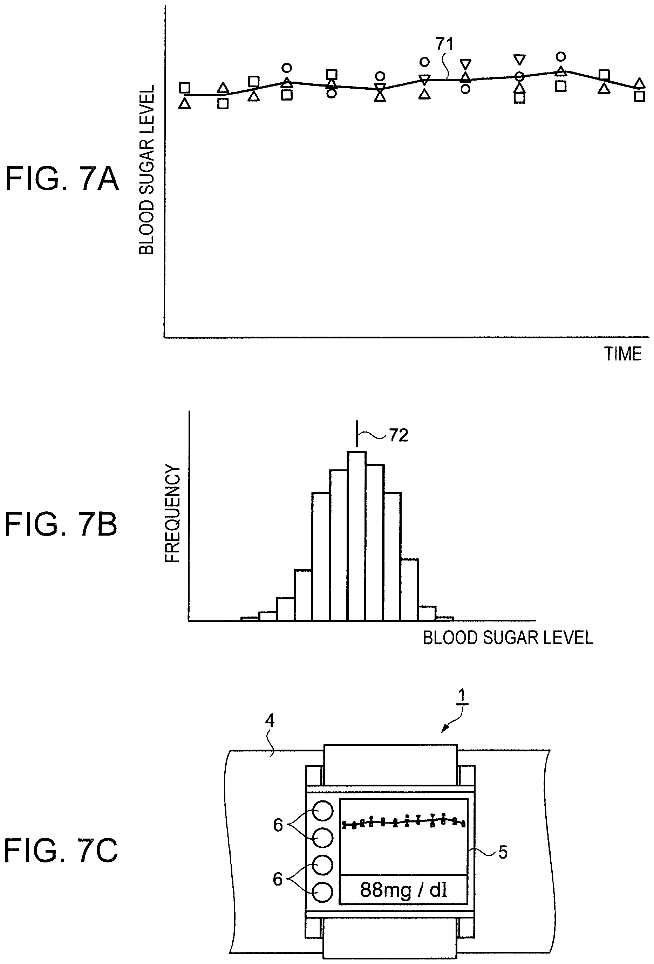

Fig. 7(a) and Fig. 7(b) are diagrams corresponding to the average calculating step in step S7. In Fig. 7(a), the ordinate indicates the measured blood sugar level. The blood sugar level is higher on the upper side than the lower side in the figure. The abscissa indicates a change of a measurement time. The time changes from the left side to the right side in the figure. A plot of circles indicates an example of measurement values of blood sugar levels calculated using spectra detected by the first infrared sensor 11. A plot of squares indicates an example of measurement values of blood sugar levels calculated using spectra detected by the second infrared sensor 13. A plot of inverted triangles indicates an example of measurement values of blood sugar levels calculated using spectra detected by the third infrared sensor 15. A plot of triangles indicates an example of measurement values of blood sugar levels calculated using spectra detected by the fourth infrared sensor 17.

The average calculating section 53 calculates a simple average using only data of the blood sugar level at the time when the blood sugar level can be calculated without using data at the time when the blood sugar level cannot be measured. When the infrared sensors of the first infrared sensor 11 to the fourth infrared sensor 17 detect spectra, the average calculating section 53 calculates an average of four measurement values. For example, when the first infrared sensor 11 and the fourth infrared sensor 17 detect spectra and the second infrared sensor 13 and the third infrared sensor 15 are not driven, the average calculating section 53 calculates an average of two measurement values. The average calculating section 53 calculates a blood sugar level transition line 71.

Fig. 7(b) is a histogram showing a distribution of blood sugar levels. The ordinate indicates a frequency. The abscissa indicates the blood sugar level. The blood sugar level is higher on the right side than the left side in the figure. Data of the blood sugar levels are blood sugar levels measured using the spectra detected by the first infrared sensor 11 to the fourth infrared sensor 17. The average calculating section 53 calculates a simple moving average. The number of data is not particularly limited. However, in this embodiment, a maximum number of data is set to fifty. When the number of data is smaller than the maximum number of data, the average calculating section 53 calculates an average of the measured data. When the number of data is equal to or larger than fifty, the average calculating section 53 calculates an average 72 of fifty data, measurement times of which are close to the present time. Therefore, the average 72 is updated when new data is measured.

Fig. 7(c) is a diagram corresponding to the display step in step S8. As shown in Fig. 7(c), in step S8, a measurement result is displayed on the display device 5. A plot of calculated blood sugar levels and the blood sugar level transition line 71 are displayed on the display device 5. Further, a value of the average close to the present time is displayed.

In the end determining step in step S9, the operator determines whether the measurement of the blood sugar level is continued. When ending the measurement of the blood sugar level, the operator pushes the input device 6 to instruct the end of the measurement of the blood sugar level. The CPU 30 inputs a signal to the input device 6 and stops program for measuring the blood sugar level. According to the steps explained above, the processing of the biological measurement method is ended.

As explained above, according to this embodiment, there are effects explained below.
(1) According to this embodiment, the first force sensor 12 to the fourth force sensor 18 detect forces of the first infrared sensor 11 to the fourth infrared sensor 17 and the arm 4 pressing each other. The pressing-force determining section 54 compares the forces with the lower determination value 46a and the upper determination value 46b and determines whether the forces are within the predetermined range.
   When the first infrared sensor 11 to the fourth infrared sensor 17 are apart from the arm 4, the infrared ray 26 is not absorbed by the arm 4. Therefore, the first infrared sensor 11 to the fourth infrared sensor 17 cannot accurately receive the infrared ray 26 that reflects information concerning the blood sugar level. When the first infrared sensor 11 to the fourth infrared sensor 17 excessively press the arm 4, the blood vessel of the arm 4 is pressed and a blood flow near the surface of the arm 4 is hindered. Consequently, since the arm 4 changes to a state unsuitable for measurement, the light intensity data received by the first infrared sensor 11 to the fourth infrared sensor 17 changes to data that does not correctly reflects the state of the blood of the arm 4.
   The pressing-force determining section 54 determines a state in which the infrared sensors of the first infrared sensor 11 to the fourth infrared sensor 17 press the arm 4. When light intensity data output by the first infrared sensor 11 to the fourth infrared sensor 17 are suitable for measurement of the blood flowing in the blood vessel of the arm 4, the component-concentration calculating section 51 calculates a blood sugar level utilizing the light intensity data. When the light intensity data output by the first infrared sensor 11 to the fourth infrared sensor 17 are unsuitable for the measurement of the blood flowing in the blood vessel of the arm 4, the component-concentration calculating section 51 does not utilize the light intensity data. Therefore, since the biological measurement apparatus 1 utilizes only light intensity data that reflects the state of the blood of the arm 4 among the light intensity data output by the first infrared sensor 11 to the fourth infrared sensor 17, it is possible to accurately measure the blood sugar level.
(2) According to this embodiment, the first infrared sensor 11 to the fourth infrared sensor 17 are set in the biological measurement apparatus 1. The infrared sensors output light intensity data. The average calculating section 53 calculates an average of the light intensity data output by the first infrared sensor 11 to the fourth infrared sensor 17. Therefore, it is possible to suppress the influence of disturbances and measure a highly reliable blood sugar value.
(3) According to this embodiment, the pressing-force determining section 54 determines a state in which the first infrared sensor 11 to the fourth infrared sensor 17 press the arm 4. The infrared-sensor control section 49 drives the infrared sensors, forces of which pressing the living organism are within the predetermined range. Therefore, the infrared-sensor control section 49 drives only the infrared sensors capable of outputting appropriate light intensity data. Therefore, the biological measurement apparatus 1 can accurately measure component concentration of the living organism utilizing only the light intensity data that reflects a state of the living organism among the light intensity data output by the plurality of infrared sensors. Further, since electric power for driving the infrared sensors is not wasted, it is possible to drive the infrared sensors while saving resources.

### (Second Embodiment)

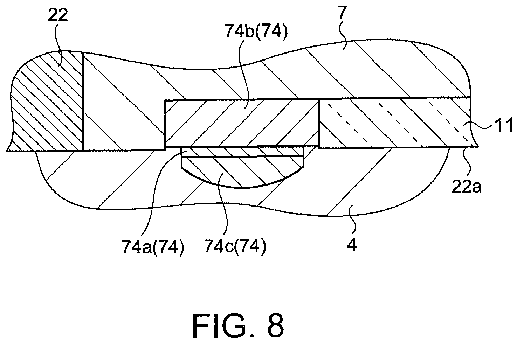

An embodiment of the biological measurement apparatus is explained with reference to a main part schematic enlarged view showing the structure of the force sensor shown in Fig. 8.

This embodiment is different from the first embodiment in that a part of the pressing protrusion 12a shown in Fig. 2(c) is an elastic body. Note that explanation is omitted concerning similarities to the first embodiment.

That is, in this embodiment, as shown in Fig. 8, a force sensor 74 includes a sensor main body section 74b. A pressing protrusion 74a is set on the arm 4 side of the sensor main body section 74b. A piezoelectric element is incorporated in the sensor main body section 74b. The piezoelectric element is held between the sensor main body section 74b and the pressing protrusion 74a. A protrusion distal end portion 74c functioning as an elastic body is set on the arm 4 side of the pressing protrusion 74a. A pressing force of the arm 4 pressing the force sensor 74 presses the force sensor 74 via the protrusion distal end portion 74c.

The material of the protrusion distal end portion 74c is an elastic body and has elasticity. The material of the protrusion distal end portion 74c only has to be an elastic body and is not particularly limited. A coil spring and the like made of metal besides silicon rubber, synthetic rubber, and natural rubber can be used. In this embodiment, for example, silicon rubber is adopted as the material of the protrusion distal end portion 74c.

As explained above, according to this embodiment, there are effects explained below.
(1) According to this embodiment, the protrusion distal end portion 74c includes the elastic body. Therefore, it is possible to prevent the arm 4 from becoming sensitive to a feeling of touch of the protrusion distal end portion 74c.

### (Third Embodiment)

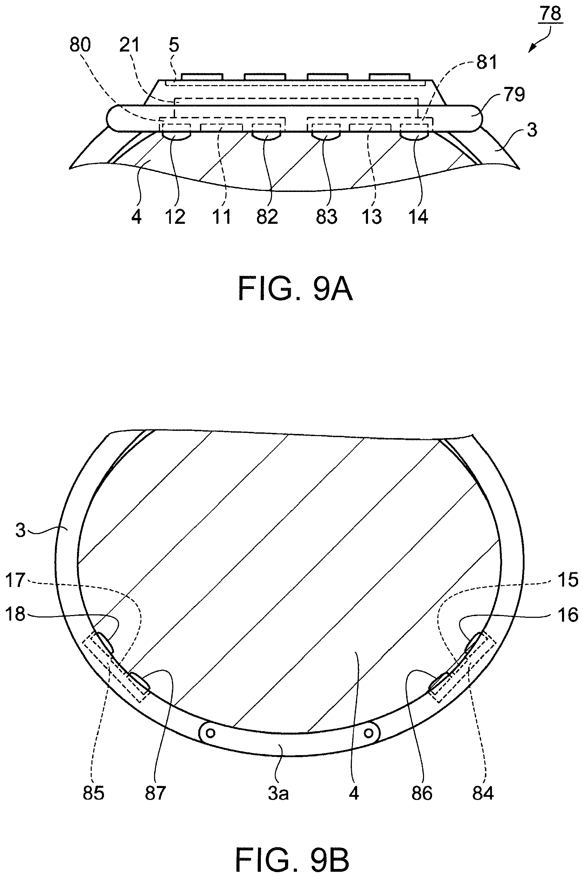

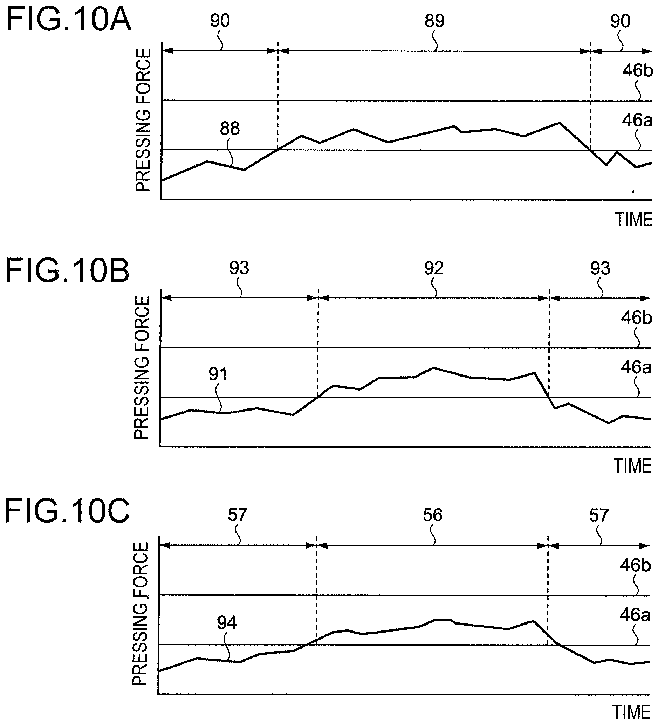

An embodiment of the biological measurement apparatus is explained with reference to Fig. 9 to Fig. 12. Fig. 9 (a) is a main part schematic enlarged view showing the structure of a main body section. Fig. 9 (b) is a main part schematic enlarged view showing the structure of a belt section. Fig. 10(a) to Fig. 10(c) are schematic diagrams for explaining a biological measurement method. This embodiment is different from the first embodiment in that a plurality of force sensors are set in a detecting section. Note that explanation is omitted concerning similarities to the first embodiment.

That is, in this embodiment, as shown in Fig. 9(a), a biological measurement apparatus 78 includes a main body section 79. A first detecting section 80 and a second detecting section 81 are set on the arm 4 side of the main body section 79. In the first detecting section 80, the first force sensor 12 and a first sub-force sensor 82 functioning as a contact-force sensor section are disposed side by side with the first infrared sensor 11. The first force sensor 12 and the first sub-force sensor 82 are disposed apart from each other in the longitudinal direction of the first infrared sensor 11. Similarly, in the second detecting section 81, the second force sensor 14 and a second sub-force sensor 83 functioning as a contact-force sensor section are disposed side by side with the second infrared sensor 13. The second force sensor 14 and the second sub-force sensor 83 are disposed apart from each other in the longitudinal direction of the second infrared sensor 13.

When the first detecting section 80 is apart from the arm 4, pressing forces detected by the first force sensor 12 and the first sub-force sensor 82 decrease. When the first detecting section 80 tilts, the pressing force detected by one of the first force sensor 12 and the first sub-force sensor 82 decreases. At this point, there is a risk that the first infrared sensor 11 separates from the arm 4. Therefore, it is possible to surely detect, using outputs of the first force sensor 12 and the first sub-force sensor 82, whether the first infrared sensor 11 is in contact with the arm 4. The second detecting section 81 has structure same as the structure of the first detecting section 80. Therefore, it is possible to surely detect, using outputs of the second force sensor 14 and the second sub-force sensor 83, whether the second infrared sensor 13 is in contact with the arm 4.

As shown in Fig. 9(b), a third detecting section 84 and a fourth detecting section 85 are set on the arm 4 side of the belt section 3. In the third detecting section 84, the third force sensor 16 and a third sub-force sensor 86 functioning as a contact-force sensor section are disposed side by side with the third infrared sensor 15. The third force sensor 16 and the third sub-force sensor 86 are disposed apart from each other in the longitudinal direction of the third infrared sensor 15. Similarly, in the fourth detecting section 85, the fourth force sensor 18 and a fourth sub-force sensor 87 functioning as a contact-force sensor section are disposed side by side with the fourth infrared sensor 17. The fourth force sensor 18 and the fourth sub-force sensor 87 are disposed apart from each other in the longitudinal direction of the fourth infrared sensor 17.

The third detecting section 84 and the fourth detecting section 85 have structure same as the structure of the first detecting section 80. Therefore, it is possible to surely detect, using outputs of the third force sensor 16 and the third sub-force sensor 86, whether the third infrared sensor 15 is in contact with the arm 4. Further, it is possible to surely detect, using outputs of the fourth force sensor 18 and the fourth sub-force sensor 87, whether the fourth infrared sensor 17 is in contact with the arm 4.

A biological measurement method using the first detecting section 80 is explained. Fig. 10(a) and Fig. 10(b) are diagrams corresponding to the pressing-force detecting step in step S2 and the force determining step in step S3. In Fig. 10(a), the ordinate indicates the pressing force detected by the first force sensor 12. The pressing force is larger on the upper side than the lower side in the figure. The abscissa indicates elapse of time. The time changes from the left to the right. A first main force transition line 88 indicates an example of transition of the pressing force detected by the first force sensor 12.

The pressing-force determining section 54 compares the pressing force detected by the first force sensor 12 and the lower determination value 46a. Further, the pressing-force determining section 54 compares the pressing force detected by the first force sensor 12 and the upper determination value 46b. Time when the pressing force is proper in the first main force transition line 88 is referred to as first main proper section 89. Time when the pressing force in the first main force transition line 88 is insufficient or excessive is referred to as first main improper section 90.

Similarly, in Fig. 10(b), the ordinate indicates the pressing force detected by the first sub-force sensor 82. The pressing force is larger on the upper side than the lower side in the figure. The abscissa is the same as the abscissa in Fig. 10 (a). Explanation of the abscissa is omitted. A first sub-force transition line 91 indicates an example of transition of the pressing force detected by the first sub-force sensor 82. The pressing-force determining section 54 compares the pressing force detected by the first sub-force sensor 82 and the lower determination value 46a. Further, the pressing-force determining section 54 compares the pressing force detected by the first sub-force sensor 82 and the upper determination value 46b. Time when the pressing force is proper in the first sub-force transition line 91 is referred to as first sub-proper section 92. Time when the pressing force is insufficient or excessive in the first sub-force transition line 91 is referred to as first sub-improper section 93.

Fig. 10(c) is a diagram corresponding to the force determining step in step S3 and the correcting step in step S5. In Fig. 10(c), the ordinate indicates the pressing force. The pressing force is larger on the upper side than the lower side in the figure. The abscissa indicates elapse of time. The time changes from the left to the right in the figure. In step S3, the pressing-force determining section 54 calculates a section, which is the first main proper section 89 and the first sub-proper section 92, and sets the section as the first proper section 56. The pressing-force determining section 54 sets a section other than the first proper section 56 as the first improper section 57. That is, when both of the pressing force detected by the first force sensor 12 and the pressing force detected by the first sub-force sensor 82 are equal to or larger than the lower determination value 46a and equal to or smaller than the upper determination value 46b, the pressing-force determining section 54 determines that the pressing force is proper. In the examples of the first main transition line 88 and the first sub-force transition line 91, the first sub-proper section 92 is included in the first main proper section 89. Therefore, the first proper section 56 is a section same as the first sub-proper section 92.

In step S5, the average calculating section 53 calculates a pressing force average, which is an average of the pressing force detected by the first force sensor 12 and the pressing force detected by the first sub-force sensor 82. A pressing force average transition line 94 is a transition line obtained by calculating an average of the first main force transition line 88 and the first sub-force transition line 91. The average calculating section 53 corrects the light intensity distribution line 68 using the pressing force average transition line 94 and the correction curve 69. That is, as the pressing force of the pressing of the first infrared sensor 11 by the arm 4, the average of the pressing force detected by the first force sensor 12 and the pressing force detected by the first sub-force sensor 82 is applied. Consequently, it is possible to accurately detect the pressing force of the pressing of the first infrared sensor 11 by the arm 4.

The second detecting section 81, the third detecting section 84, and the fourth detecting section 85 have structure same as the structure of the first detecting section 80. In step S3, the pressing-force determining section 54 calculates proper sections and improper sections of the second detecting section 81, the third detecting section 84, and the fourth detecting section 85 with a method same as the method with which the pressing-force determining section 54 calculates the first proper section 56 and the first improper section 57.

In step S5, the average calculating section 53 calculates pressing force average transition lines of the second detecting section 81, the third detecting section 84, and the fourth detecting section 85 with a method same as the method with which the average calculating section 53 calculates the pressing force average transition line 94. The average calculating section 53 corrects the light intensity distribution line 68 using the calculated pressing force average transition lines and the correction curve 69. The following steps are the same as the steps in the first embodiment. Explanation of the steps is omitted.

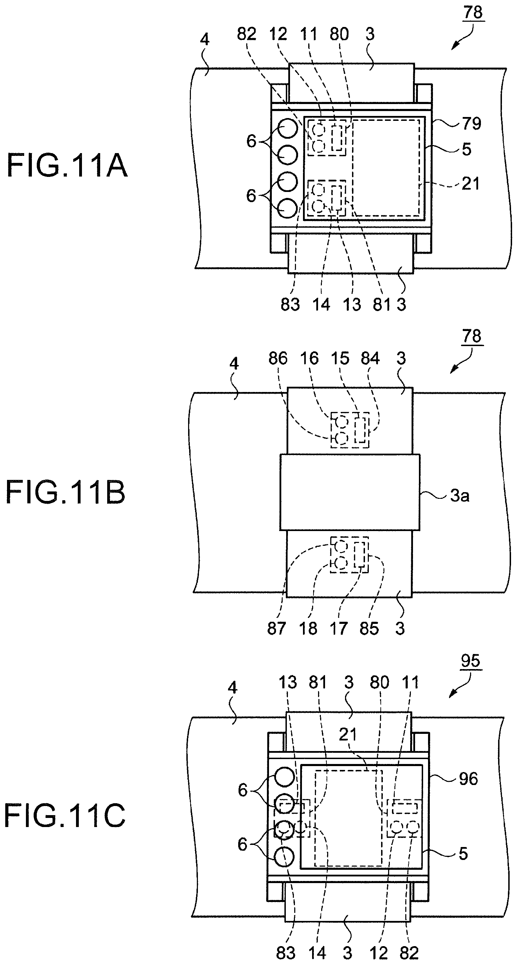

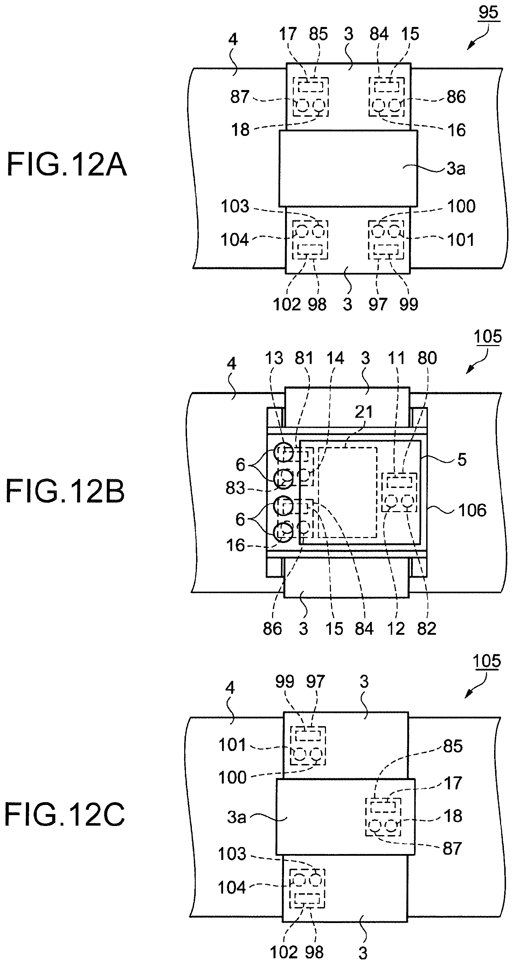

Fig. 11 and Fig. 12 are schematic diagrams showing dispositions of the detecting section. In Fig. 11(a), the first detecting section 80 and the second detecting section 81 are set side by side in a direction orthogonal to the longitudinal direction of the arm 4 in the main body section 79 of the biological measurement apparatus 78. In the first detecting section 80, the first force sensor 12 and the first sub-force sensor 82 are disposed side by side in the direction orthogonal to the longitudinal direction of the arm 4. In this form, it is possible to detect a state in which the first detecting section 80 tilts with the longitudinal direction of the arm 4 set as a rotation axis. Similarly, in the second detecting section 81, the second force sensor 14 and the second sub-force sensor 83 are disposed side by side in the direction orthogonal to the longitudinal direction of the arm 4. In this form, it is possible to detect a state in which the second detecting section 81 inclines with the longitudinal direction of the arm 4 set as a rotation axis.

In Fig. 11 (b), the third detecting section 84 and the fourth detecting section 85 are disposed side by side in the direction orthogonal to the longitudinal direction of the arm 4 in the belt section 3 of the biological measurement apparatus 78. In the third detecting section 84, the third force sensor 16 and the third sub-force sensor 86 are disposed side by side in the direction orthogonal to the longitudinal direction of the arm 4. In this form, it is possible to detect a state in which the third detecting section 84 tilts with the longitudinal direction of the arm 4 set as a rotation axis. Similarly, in the fourth detecting section 85, the fourth force sensor 18 and the fourth sub-force sensor 87 are disposed side by side in the direction orthogonal to the longitudinal direction of the arm 4. In this form, it is possible to detect a state in which the fourth detecting section 85 tilts with the longitudinal direction of the arm 4 set as a rotation axis.

A biological measurement apparatus 95 different from the biological measurement apparatus 78 in the disposition of detectors is explained. In Fig. 11(c), in a main body section 96 of the biological measurement apparatus 95, the first detecting section 80 and the second detecting section 81 are disposed side by side in the longitudinal direction of the arm 4 across the control section 21. In the first detecting section 80, the first force sensor 12 and the first sub-force sensor 82 are disposed side by side in the longitudinal direction of the arm 4. In this form, it is possible to detect a state in which the first detecting section 80 tilts with the direction orthogonal to the longitudinal direction of the arm 4 set as a rotation axis. Similarly, in the second detecting section 81, the second force sensor 14 and the second sub-force sensor 83 are disposed side by side in the longitudinal direction of the arm 4. In this form, it is possible to detect a state in which the second detecting section 81 tilts with the direction orthogonal to the longitudinal direction of the arm 4 set as a rotation axis.

In Fig. 12 (a), the third detecting section 84 and the fourth detecting section 85 are set on the upper side in the figure of the adjusting section 3a in the belt section 3 of the biological measurement apparatus 95. A fifth detecting section 97 functioning as a detecting section and a sixth detecting section 98 functioning as a detecting section are set on the lower side in the figure of the adjusting section 3a.

The third detecting section 84 and the fourth detecting section 85 are set side by side in the longitudinal direction of the arm 4. In the third detecting section 84, the third force sensor 16 and the third sub-force sensor 86 are disposed side by side in the longitudinal direction of the arm 4. In this form, it is possible to detect a state in which the third detecting section 84 tilts with the direction orthogonal to the longitudinal direction of the arm 4 set as a rotation axis. Similarly, in the fourth detecting section 85, the fourth force sensor 18 and the fourth sub-force sensor 87 are disposed side by side in the longitudinal direction of the arm 4. In this form, it is possible to detect a state in which the fourth detecting section 85 tilts with the direction orthogonal to the longitudinal direction of the arm 4 set as a rotation axis.

The fifth detecting section 97 and the sixth detecting section 98 are set side by side in the longitudinal direction of the arm 4. A fifth infrared sensor 99 functioning as an infrared sensor section, a fifth force sensor 100 functioning as a contact-force sensor section, and a fifth sub-force sensor 101 functioning as a contact-force sensor section are set in the fifth detecting section 97. The fifth infrared sensor 99 is a sensor same as the first infrared sensor 11. The fifth force sensor 100 and the fifth sub-force sensor 101 are sensors same as the first force sensor 12. The fifth force sensor 100 and the fifth sub-force sensor 101 are disposed side by side in the longitudinal direction of the arm 4. In this form, it is possible to detect a state in which the fifth detecting section 97 tilts with the direction orthogonal to the longitudinal direction of the arm 4 set as a rotation axis.

Similarly, a sixth infrared sensor 102 functioning as an infrared sensor section, a sixth force sensor 103 functioning as a contact-force sensor section, and a sixth sub-force sensor 104 functioning as a contact-force sensor section are set in the sixth detecting section 98. The sixth infrared sensor 102 is a sensor same as the first infrared sensor 11. The sixth force sensor 103 and the sixth sub-force sensor 104 are sensors same as the first force sensor 12. The sixth force sensor 103 and the sixth sub-force sensor 104 are disposed side by side in the longitudinal direction of the arm 4. In this form, it is possible to detect a state in which the sixth detecting section 98 tilts with the direction orthogonal to the longitudinal direction of the arm 4 set as a rotation axis.

When the biological measurement apparatus 95 tilts with the direction orthogonal to the longitudinal direction of the arm 4 set as a rotation axis, one detecting section of the first detecting section 80 and the second detecting section 81 can detect a blood sugar level. Further, one detecting section of the third detecting section 84 and the fourth detecting section 85 can detect a blood sugar level. Further, one detecting section of the fifth detecting section 97 and the sixth detecting section 98 can detect a blood sugar level. When the arm 4 performs a twisted motion, any one detecting section among the first detecting section 80 to the sixth detecting section 98 can detect a blood sugar level. Therefore, the biological measurement apparatus 95 can increase robustness.

A biological measurement apparatus 105 different from the biological measurement apparatus 78 and the biological measurement apparatus 95 in the disposition of detectors is explained. In Fig. 12(b), the first detecting section 80, the second detecting section 81, and the third detecting section 84 are set in a main body section 106 of the biological measurement apparatus 105. The first detecting section 80, the second detecting section 81, and the third detecting section 84 are disposed on a concentric circle centering on the control section 21. An angle formed by the first detecting section 80 and the second detecting section 81 centering on the control section 21 is 120 degrees. An angle formed by the first detecting section 80 and the third detecting section 84 centering on the control section 21 is 120 degrees.

In this state, it is possible to detect, using the second detecting section 81 and the third detecting section 84, a state in which the main body section 106 tilts with the longitudinal direction of the arm 4 set as a rotation axis. Further, it is possible to detect, using the first detecting section 80 to the third detecting section 84, a state in which the main body section 106 tilts with the direction orthogonal to the longitudinal direction of the arm 4 set as a rotation axis. Therefore, even when the main body section 106 tilts in any direction, it is possible to detect that the main body section 106 tilts.

In Fig. 12 (c), in the biological measurement apparatus 105, the fourth detecting section 85 is disposed on the right side in the figure in the adjusting section 3a. The fifth detecting section 97 is set on the upper side in the figure of the adjusting section 3a in the belt section 3. The sixth detecting section 98 is set on the lower side in the figure of the adjusting section 3a. The fifth detecting section 97 and the sixth detecting section 98 are located on the left side in the figure in the belt section 3.

In the fourth detecting section 85, the fourth force sensor 18 and the fourth sub-force sensor 87 are disposed side by side in the longitudinal direction of the arm 4. In the fifth detecting section 97, the fifth force sensor 100 and the fifth sub-force sensor 101 are disposed side by side in the longitudinal direction of the arm 4. In the sixth detecting section 98, the sixth force sensor 103 and the sixth sub-force sensor 104 are disposed side by side in the longitudinal direction of the arm 4. In this form, it is possible to detect a state in which the fourth detecting section 85, the fifth detecting section 97, and the sixth detecting section 98 tilt with the direction orthogonal to the longitudinal direction of the arm 4 set as a rotation axis.

When the biological measurement apparatus 105 tilts with the direction orthogonal to the longitudinal direction of the arm 4 set as a rotation axis, any one detecting section among the first detecting section 80 to the sixth detecting section 98 can detect a blood sugar level. Further, when the arm 4 performs a twisted motion, any one detecting section among the first detecting section 80 to the sixth detecting section 98 can detect a blood sugar level. Therefore, the biological measurement apparatus 105 can increase robustness.

### (Fourth Embodiment)

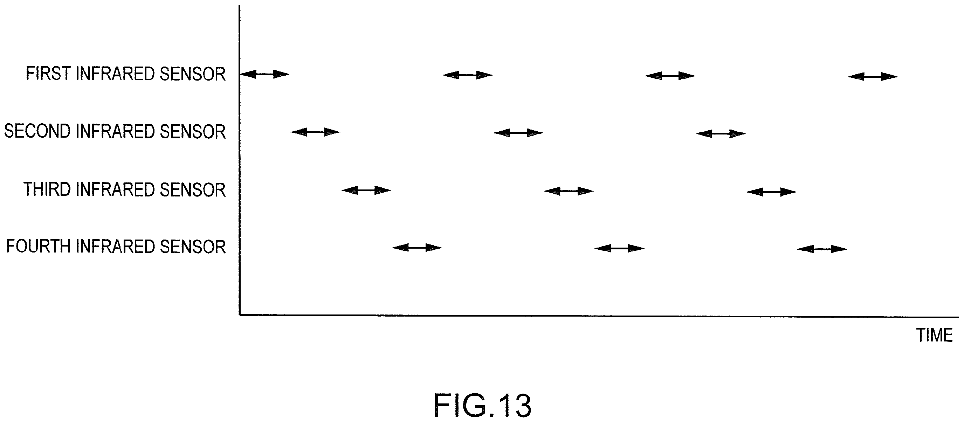

An embodiment of the biological measurement apparatus is explained with reference to Fig. 13. Fig. 13 is a time chart showing driving timings of infrared sensors. This embodiment is different from the first embodiment in that detecting sections sequentially perform measurement. Note that explanation is omitted concerning similarities to the first embodiment.

In Fig. 13, the ordinate indicates the first infrared sensor 11 to the fourth infrared sensor 17. The abscissa indicates a change of time. In the figure, the time changes from the left side to the right side. Arrow lines indicate timings when the sensors are driven. The first infrared sensor 11 to the fourth infrared sensor 17 are driven in the infrared-ray detecting step in step S4. At this point, first, the first infrared sensor 11 is driven. When the driving of the first infrared sensor 11 ends, subsequently, the second infrared sensor 13 is driven. When the driving of the second infrared sensor 13 ends, subsequently, the third infrared sensor 15 is driven. When the driving of the third infrared sensor 15 ends, subsequently, the fourth infrared sensor 17 is driven. When the driving of the fourth infrared sensor 17 ends, subsequently, the first infrared sensor 11 is driven. In this way, the first infrared sensor 11 to the fourth infrared sensor 17 are sequentially driven.

In parallel to time when one infrared sensor is driven, calculation is performed using a detection result of the infrared sensor. That is, step S4 and step S5 to step S8 are performed in parallel.

As explained above, according to this embodiment, there are effects explained below.
(1) According to this embodiment, the infrared sensors are sequentially driven. Consequently, compared with when the plurality of infrared sensors are simultaneously driven, it is possible to reduce a peak value of electric power for driving the infrared sensors. Therefore, since an ability to supply the electric power can be reduced, it is possible to reduce the capacity of a power supply.

Note that this embodiment is not limited to the embodiments explained above. Various changes and improvements can also be added by those having ordinary knowledge in the field within the technical idea of the present invention. Modifications are explained below.

### (Modification 1)

In the first embodiment, the first light receiving device 25 has the function of splitting the infrared ray 26. The present invention is not limited to this. The first light emitting device 24 may radiate the infrared ray 26 having the predetermined wavelength. The first light receiving device 25 may detect light intensity without splitting the infrared ray 26. For example, the first light emitting device 24 may include a plurality of LEDs having different wavelengths that emit lights. The LED to emit light may be switched to switch the wavelength of the infrared ray 26 and cause the LED to emit light. Further, the wavelength may be shifted using a diffraction grating. The infrared ray 26 having the predetermined wavelength may be emitted using various methods.

### (Modification 2)

In the first embodiment, the intensity distribution of the infrared ray 26 with respect to the wavelength of the first light emitting device 24 is not taken into account. The correction calculating section 52 may correct data of the light intensity distribution line 68 detected by the first infrared sensor 11 according to the intensity distribution of the infrared ray 26 with respect to the wavelength of the first light emitting device 24. It is possible to improve accuracy of a measurement value of a blood component.

### (Modification 3)

In the first embodiment, the first light receiving device 25 outputs the data of the light intensity distribution line 68. The first light receiving device 25 may detect only light intensity of the predetermined wavelength. By reducing types of wavelengths of the infrared ray 26 to be detected, the first light receiving device 25 can output the light intensity of the predetermined wavelength in a short time.

### (Modification 4)

In the first embodiment, the control section 21 is housed on the inside of the main body section 2. When the control section 21 cannot be fully housed on the inside of the main body section 2, a part of or the entire control section 21 may be housed in an electronic device separate from the main body section 2. The electronic device and the main body section 2 may communicate by wire or radio. This makes it easy to manufacture the biological measurement apparatus 1.

### (Modification 5)

In the first embodiment, the blood sugar level is measured. The wavelength of the infrared ray 26 to be analyzed may be changed to measure other component concentration. Besides, the infrared ray 26 may be radiated on a plurality of places to detect a pulse wave of a blood flow and measure a pulse rate and blood pressure.

### (Modification 6)

In the first embodiment, the infrared-sensor control section 49 drives the first infrared sensor 11 only in the first proper section 56. The first infrared sensor 11 may be always driven. Only the light intensity data 44 in the first proper section 56 may be adopted. It is possible to facilitate the control of the first infrared sensor 11.

### (Modification 7)

In the first embodiment, the correction calculating section 52 corrects the light intensity distribution line 68. The correction may be omitted when the influence of the correction is small. It is possible to reduce data of the program software 43. Note that the contents of the modification 1 to the modification 7 may be applied to the second embodiment to the fourth embodiment as well.

### (Modification 8)

In the third embodiment, each of the first detecting section 80 to the sixth detecting section 98 includes the two force sensors. The number of force sensors included in each of the detecting sections may be three or more. Further, it is possible to accurately detect a state in which the detecting sections are in contact with the arm 4.

### (Modification 9)

In the third embodiment, the direction in which the force sensors are disposed side by side in the detectors is aligned. The direction in which the force sensors are disposed side by side may be changed for each of the detecting sections. The force sensors may be disposed according to a form in which the belt section 3 is easily deformed. Since the force sensors can easily detect the tilts of the detecting sections, it is possible to accurately measure biological information.

### Reference Signs List

1, 78, 95, 105 biological measurement apparatus
2 main body section functioning as a holding section
3 belt section functioning as a holding section
4 arm serving as a living organism
7 first detecting section functioning as a detecting section
8 second detecting section functioning as a detecting section
9 third detecting section functioning as a detecting section
10 fourth detecting section functioning as a detecting section
11 first infrared sensor functioning as an infrared sensor section
12 first force sensor functioning as a contact-force sensor section
13 second infrared sensor functioning as an infrared sensor section
14 second force sensor functioning as a contact-force sensor section
15 third infrared sensor functioning as an infrared sensor section
16 third force section functioning as a contact-force sensor section
17 fourth infrared sensor functioning as an infrared sensor section
18 fourth force sensor functioning as a contact-force sensor section
26 infrared ray
51 component-concentration calculating section
53 average calculating section
54 pressing-force determining section functioning as a determining section
74 force sensor
82 first sub-force sensor functioning as a contact-force sensor section
83 second sub-force sensor functioning as a contact-force sensor section
86 third sub-force sensor functioning as a contact-force sensor section
87 fourth sub-force sensor functioning as a contact-force sensor section
97 fifth detecting section functioning as a detecting section
98 sixth detecting section functioning as a detecting section
99 fifth infrared sensor functioning as an infrared sensor section
100 fifth force sensor functioning as a contact-force sensor section
101 fifth sub-force sensor functioning as a contact-force sensor section
102 sixth infrared sensor functioning as an infrared sensor section
103 sixth force sensor functioning as a contact-force sensor section
104 sixth sub-force sensor functioning as a contact-force sensor section

## Claims

1. A biological measurement apparatus comprising:
a plurality of detecting sections including an infrared sensor section that radiates an infrared ray on a living organism, receives the infrared ray reflected or scattered on the living organism, and outputs light intensity data and a contact-force sensor section that detects a contact force between the infrared sensor section and the living organism;
a holding section that holds the detecting section such that the infrared sensor section is in contact with the living organism;
a determining section that compares the contact force with a determination value and determines whether the contact force is within a predetermined range; and
a component-concentration calculating section that calculates component concentration of the living organism using the light intensity data at a time when the determining section determines that the contact force is within the predetermined range.

2. The biological measurement apparatus according to claim 1, further comprising an average calculating section that calculates an average of the component concentration calculated from the light intensity data output by a plurality of the infrared sensor sections.

3. The biological measurement apparatus according to claim 1 or 2, wherein a plurality of the infrared sensor sections are sequentially driven.

4. The biological measurement apparatus according to any one of claims 1 to 3, wherein the detecting section includes a plurality of the contact-force sensor sections.

5. A biological measurement apparatus comprising:
a plurality of detecting sections including an infrared sensor section that radiates an infrared ray on a living organism, receives the infrared ray reflected or scattered on the living organism, and outputs light intensity data and a contact-force sensor section that detects a contact force between the infrared sensor section and the living organism;
a holding section that holds the detecting section such that the infrared sensor section is in contact with the living organism;
a determining section that compares the contact force with a determination value and determines whether the contact force is within a predetermined range;
an infrared-sensor control section that performs control for driving the infrared sensor section when the contact force is within a predetermined range; and
a component-concentration calculating section that calculates component concentration of the living organism using the light intensity data.

6. A biological measurement method comprising:
pressing a plurality of infrared sensor sections against a living organism;
detecting contact forces between the infrared sensor sections and the living organism;
driving the infrared sensor sections, the contact forces of which are within a predetermined range;
the infrared sensor sections radiating infrared ray on the living organism, receiving the infrared ray reflected or scattered on the living organism, and outputting light intensity data; and
calculating component concentration of the living organism from the light intensity data.
